# EUROPÄISCHE PATENTANMELDUNG

(11) **EP 1 103 536 A1**
(43) Veröffentlichungstag der Anmeldung: **30.05.2001**
(21) Anmeldenummer: 00123091.1
(22) Anmeldetag: 25.10.2000
(51) Int. Cl.: C07B 53/00, C07C 209/70, C07D 295/023, C07D 295/03, C07D 209/08

(54) **Verfahren zur Herstellung von chiralen Aminen durch asymmetrische Hydrierung von Enaminen**

(30) Priorität: 24.11.1999 DE 19956414
(71) Anmelder: Aventis Research & Technologies GmbH & Co. KG, 65926 Frankfurt am Main (DE)
(72) Erfinder: Riermeier, Thomas, Dr., 65439 Flörsheim (DE); Bosch, Boris E., Dr., 48161 Münster (DE); Dingerdissen, Uwe, Dr., 64342 Seeheim-Jugenheim (DE); Börner, Armin, Dr., 18055 Rostock (DE); Tararov, Vitali, Dr, Moskau 113525 (RU); Kadyrov, Renat, Dr., 18055 Rostock (DE)
(74) Vertreter: Ackermann, Joachim, Dr.

(57) **Zusammenfassung**

Gegenstand der Erfindung ist ein Verfahren zur Herstellung von chiralen Aminen durch Umsetzung von elektronenreichen Enaminen in Gegenwart von Wasserstoff und eines chiralen Katalysators umfassend mindestens einen Metall-Ligand-Komplex, der mindestens ein Metallatom ausgewählt aus der 8. Nebengruppe und ein oder mehrere mono- oder bidentate phosphorhaltige Liganden der allgemeinen Formeln (III) oder (IV) enthält,

(R⁶Y¹)(R⁷Y¹)P¹X¹ZX²P²(Y²R⁸)(Y²R⁹) (III)

(R⁶Y¹)(R⁷Y¹)(R⁸Y¹)P (IV)

## Beschreibung

Die Erfindung beschreibt die Herstellung von Aminen durch die Hydrierung von Enaminen in Gegenwart von Wasserstoff in Anwesenheit chiraler
Metallkatalysatoren unter milden Bedingungen.

Enantiomerenreine Amine spielen eine dominierende Rolle in zahlreichen komplexen Naturstoffen wie beispielsweise den Alkaloiden, Vitaminen oder Aminosäuren, deren chemische, pharmazeutische und industrielle Bedeutung unbestritten ist. Als chemische Zwischenprodukte finden Amine u. a. Anwendung in der Synthese von Pharmazeutika, Agrochemikalien, Nahrungsmittelzusatzstoffen, Farbstoffen oder Kosmetika.

Eine Methode zur Synthese enantiomerenreiner Amine ist die enantioselektive Hydrierung von N-acylierten elektronenarmen Enamiden. So ist die asymmetrische Hydrierung von *N*-Acyl-dehydroaminosäuren eine wichtige Methode zur Herstellung von optisch aktiven *N*-Acyl-aminosäuren (Catalytic Asymmetric Synthesis, VCH, New York, 1993, 1 ff; Chirotechnology, Marcel Dekker, Inc., New York, 1993, 287ff). Insbesondere die Hydrierung mit Rh(I)-Katalysatoren auf der Basis von phosphorhaltigen Liganden wurde zu einer leistungsfähigen Methode entwickelt, die die gewünschten Aminosäuren in teilweise sehr guten Enantioselektivitäten liefert (z. B. H. W. Kreuzfeld et al. Amino Acids, 1996, 11, 269; RajanBabu et al. J. Org. Chem. 1999, 64, 3429; Yamanoi et al. J. Org. Chem. 1999, 64, 2988; Chen et al. Tetrahedron Lett. 1999, 40, 957; Reetz et al. *Angew. Chem. Int. Ed. Engl.* 1999, *38*, 179; Kang et al. Tetrahedron Lett. 1998, 39, 5523; Almena-Perea et al. Tetrahedron Lett. 1998, 39, 8073; Reetz et al. Chem. Commun. 1998, 2077; Holz et al. J. Org. Chem. 1998, 63, 8031).

Auch elektronenarme N-Acyl-enamide ohne weitere elektronenziehende funktionelle Gruppe an der olefinischen Doppelbindung können ebenfalls katalytisch hydriert werden (Burk et al. J. Org. Chem. 1998, 63, 6084; Kagan et al. J. Orgamet. Chem. 1975, 90, 353; Sinou et al. J. Organomet. Chem. 1976, 114, 325; Morimoto et al. Chem. Pharm. Bull. 1992, 40, 2894; Zhu et al. J. Org. Chem. 1998, 63, 9590).
Es wird angenommen, daß die Bildung eines 5-gliedrigen Chelatringes zwischen N-Acyl-enamidfragment und Metall von besonderer Bedeutung für den Ablauf der Reaktion ist (Asymmetric Synthesis, Academic Press, Orlando, 1985, 41 ff). Die Herstellung derartig modifizierter Substrate erfordert zusätzliche Syntheseschritte.
Nach erfolgter Reduktion muß ebenfalls noch die Acylschutzgruppe entfernt werden, um zum gewünschten Amin zu gelangen.
Von besonderem Vorteil ist deshalb die Reduktion von unmodifizierten Aminvorstufen. Ein Beispiel dafür ist die asymmetrische Hydrierung von Iminen mit Übergangsmetallkatalysatoren (Kobayashi et al. Chem. Rev. 1999, 99, 1069; B. R. James, Catalysis Today, 1997, 37, 209). Aufgrund der geringen Reaktivität dieser Substrate müssen jedoch oftmals extreme Reaktionsbedingungen (Drucke bis zu 200 bar, Temperaturen bis zu 100 °C) angewandt werden. Mit vielen Iminen gelingt die homogene übergangsmetallkatalysierte Hydrierung überhaupt nicht (Yurovskaya et al. Tetrahedron: Asymmetry 1998, 9, 3331).
Eine Alternative stellt die Reduktion von elektronenreichen Enaminen dar. Mit der Ausnahme von Titanocen-Komplexen, die jedoch besonders drastische, technisch wenig praktikable Reaktionsbedingungen erfordern und eine nur geringe Toleranz gegenüber funktionellen Gruppen aufweisen (Buchwald et al. J. Am. Chem. Soc. 1994, 116, 5985, Buchwald et al. US 5489682; Buchwald et al. US 5489682; Buchwald et al. WO 9502567), ist nicht bekannt, daß auch andere
Homogenkatalysatoren diese Reaktion katalysieren.

Aufgabe der vorliegenden Erfindung war es daher, eine Methode zu entwickeln, die die asymmetrische Hydrierung von elektronenreichen Enaminen unter milden Bedingungen mittels Anwendung chiraler Katalysatoren ermöglicht.

Es wurde überraschenderweise gefunden, daß Übergangsmetallkomplexe der 8. Nebengruppe auf der Basis von phosphorhaltigen Liganden sehr effizient die asymmetrische Hydrierung von elektronenreichen Enaminen unter milden Bedingungen katalysieren.

Die angewandten Übergangsmetallkatalysatoren liefern in der asymmetrischen Hydrierung gute bis sehr gute Ausbeuten an gewünschtem Amin, bei Einsatz geringer Katalysatormengen. Gleichzeitig können beachtliche Enantioselektivitäten in der Hydrierung realisiert werden. Ein einfaches Verfahren ermöglicht darüber hinaus das Überführen von schwer hydrierbaren Iminen in Enamine. Somit sind diese Substanzen ebenfalls einfach in die gewünschten Amine überführbar.

Mit dem erfindungsgemäßen Verfahren werden die bekannten Nachteile der asymmetrischen Hydrierung von *N*-Acyl-enamiden bzw. der Ti-katalysierten Hydrierung von Enaminen umgangen. Gleichzeitig können reaktionsträge Imine durch Überführung in Enamine in leicht hydrierbare Substrate umgewandelt werden.

Gegenstand der Erfindung ist ein Verfahren zur Herstellung von chiralen Aminen, insbesondere von chiralen Aminen der Formel (II), durch Umsetzung von elektronenreichen Enaminen, insbesondere von Enaminen der Formel (I), 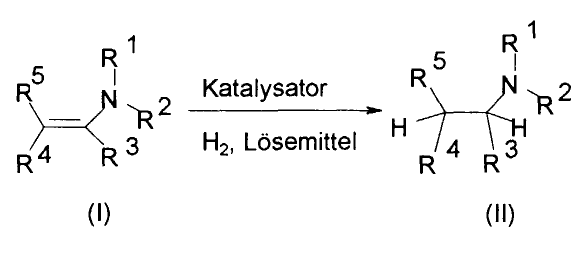 worin
- R¹ und R²: gleich oder verschieden Wasserstoff, eine C₁-C₅₀ kohlenstoffhaltige Gruppe wie C₁-C₂₄-Alkyl, C₂-C₂₀-Alkenyl, C₃-C₈-Cycloalkyl, C₅-C₈-Cycloalkenyl, wobei der Cyclus auch 1-2 Heteroatome enthalten kann, bevorzugt ausgewählt aus der Gruppe N,O, S und die Ringgröße bevorzugt 5-7 beträgt, Phenyl, Naphtyl, Fluorenyl, C₆-C₁₄-Aryl, C₂-C₁₃-Heteroaryl, wobei die Zahl der Heteroatome 1-4 betragen kann, bevorzugt ausgewählt aus der Gruppe N,O, S, und die Ringgröße bevorzugt 5 - 7 beträgt,
wobei diese Gruppen selbst jeweils ein- oder mehrfach substituiert sein können und diese Substituenten dabei unabhängig voneinander Wasserstoff, C₁-C₂₀-Alkyl, C₂-C₂₀-Alkenyl, C₁-C₁₀-Haloalkyl, C₃-C₈-Cycloalkyl, C₃-C₈-Cycloalkenyl, Phenyl, C₆-C₁₄-Aryl, C₂-C₁₃-Heteroaryl, wobei die Zahl der Heteroatome 1-4 betragen kann, bevorzugt ausgewählt aus der Gruppe N,O, S, und die Ringgröße bevorzugt 5 - 7 beträgt, C₁-C₁₀-Alkoxy, C₁-C₉-Trihalomethylalkyl, Trifluormethyl, Trichlormethyl, Fluoro, Chloro, Bromo, lodo, Nitro, Trifluormethylsulfonato, C₁-C₆-Trialkylsilyl sein können,
- R³ bis R⁵: gleich oder verschieden ein Wasserstoffatom oder ein Halogen (Fluor, Chlor, Brom, Jod) oder eine C₁-C₅₀ kohlenstoffhaltige Gruppe wie C₁-C₂₄-Alkyl, C₂-C₂₀-Alkenyl, C₃-C₈-Cycloalkyl, C₃-C₈-Cycloalkenyl, wobei der Cyclus auch 1-2 Heteroatome enthalten kann, bevorzugt ausgewählt aus der Gruppe N,O, S und die Ringgröße bevorzugt 5 - 7 beträgt, Phenyl, Naphtyl, Fluorenyl, C₆-C₁₄-Aryl, C₂-C₁₃-Heteroaryl, wobei die Zahl der Heteroatome 1 - 4 betragen kann, bevorzugt ausgewählt aus der Gruppe N,O, S, und die Ringgröße bevorzugt 5-7 beträgt,
wobei diese Gruppen selbst jeweils ein- oder mehrfach substituiert sein können und diese Substituenten dabei unabhängig voneinander Wasserstoff, C₁-C₂₀-Alkyl, C₂-C₂₀-Alkenyl, C₁-C₁₀-Haloalkyl, C₃-C₈-Cycloalkyl, C₃-C₈-Cycloalkenyl, wobei der Cyclus auch 1 - 2 Heteroatome enthalten kann, bevorzugt ausgewählt aus der Gruppe N,O, S und die Ringgröße bevorzugt 5 - 7 beträgt, Phenyl, C₆-C₁₄-Aryl, C₃-C₁₃-Heteroaryl, wobei die Zahl der Heteroatome 1 - 4 betragen kann, bevorzugt ausgewählt aus der Gruppe N,O, S, und die Ringgröße bevorzugt 5 - 7 beträgt, C₁-C₁₀-Alkoxy, C₁-C₉-Trihalomethylalkyl, Trifluormethyl, Trichlormethyl, Fluoro, Chloro, Bromo, lodo, Nitro, Hydroxy, Trifluormethylsulfonato, Thio, Thiolato, Amino, C₁-C₈-substituierte Amino der Formen NH₂, NH-Alkyl-C₁-C₈, NH-Aryl-C₅-C₆, N-Alkyl₂-C₁-C₈, N-Aryl₂-C₅-C₆, N-Alkyl₃-C₁-C₈ ⁺, N-Aryl₃-C₅-C₆ ⁺, NH-CO-Alkyl-C₁-C₈, NH-CO-Aryl-C₅-C₆, Cyano, C₁-C₆-Acyloxy, Sulfinato, Sulfonato der Formen SO₃H und SO₃R¹², Phosphato der Formen PO₃H₂, PO₃HR¹² und PO₃ R¹² ₂ wobei R¹² entweder ein einwertiges Kation, NH₄ ⁺, N(Alkyl)₄ ⁺, N(Aryl)₄ ⁺, C₁-C₈-Alkyl oder C₆-Aryl darstellt, C₁-C₆-Trialkylsilyl, sein können, und
dabei können R¹ bis R⁵ durch eine und oder mehrere kovalente Bindungen verknüpft sein, so daß ein cyclisches System vorliegt,
in Gegenwart von Wasserstoff und eines chiralen Katalysators umfassend mindestens einen Metall-Ligand-Komplex, der mindestens ein Metallatom ausgewählt aus der 8. Nebengruppe und ein oder mehrere mono- oder bidentate achirale oder chirale Liganden der allgemeinen Formeln (III) oder (IV) enthält,

(R⁶Y¹)(R⁷Y¹)P¹X¹ZX²P²(Y²R⁸)(Y²R⁹) (III)

(R⁶Y¹)(R⁷Y¹)(R⁸Y¹)P (IV)

wobei
- R⁶ bis R⁹: gleich oder verschieden sind und ein Wasserstoffatom oder eine C₁-C₅₀ kohlenstoffhaltige Gruppe, wie z. B. C₁-C₂₄Alkyl, C₂-C₂₀ Alkenyl, C₃-C₈Cycloalkyl, C₅-C₈Cycloalkenyl, C₆-C₁₄ Aryl, Phenyl, Naphthyl, Fluorenyl, C₂-C₁₃ Heteroaryl, wobei die Zahl der Heteroatome, bevorzugt aus der Gruppe N, O, S, 1- 4 betragen kann, wobei die cyclischen aliphatischen oder aromatischen Reste bevorzugt 5 bis 7 gliedrige Ringe sind, und bei denen alle vorgenannten Substituenten jeweils ein- oder mehrfach substituiert sein können, diese Substituenten können dabei unabhängig voneinander Wasserstoff, C₁-C₂₀ Alkyl, C₂-C₂₀ Alkenyl, C₁-C₁₀ Haloalkyl, C₃-C₈ Cycloalkyl, C₅-C₈ Cycloalkenyl, C₂-C₉ Heterocycloalkyl, C₁-C₉ Heterocycloalkenyl, C₆-C₁₄Aryl, Phenyl, C₂-C₁₃ Heteroaryl, wobei die Zahl der Heteroatome, bevorzugt aus der Gruppe N, O, S, 1 - 4 betragen kann, C₁-C₁₀ Alkoxy, OCO-Alkyl-(C₁-C₈), O-Aryl-(C₅-C₁₀), 0-Phenyl, C₁-C₉ Trihalomethylalkyl, Fluoro, Chloro, Bromo, lodo, Nitro, Hydroxy, Trifluormethylsulfonato, Oxo, Thio, Thiolato, Amino, C₁-C₈ substituierte Amino der Formen mono-, di-, tri- C₁-C₈-Alkylamino oder C₂-C₈ Alkenylamino oder mono-, di-, tri- C₆-C₈ Arylamino oder C₁-C₈-Alkyl-C₆-C₈-arylamino, NH-CO-Alkyl- C₁-C₈, NH-CO-Aryl- C₆-C₈, Cyano, C₁-C₈-Acyloxy, Carboxyl, Carboxylato der Form COOR¹², Sulfinato, Sulfonato der Form SO₃R¹², Phosphato der Form PO₃H₂, PO₃HR¹², PO₃R¹² ₂, wobei wobei R¹² entweder ein einwertiges Kation, NH₄ ⁺, N(Alkyl)₄ ⁺, N(Aryl)₄ ⁺, C₁-C₈-Alkyl oder C₆-Aryl darstellt, Tri- C₁-C₆ Alkylsilyl, sein, und wobei zwei dieser Substituenten auch verbrückt sein können,
und R⁶ und R⁷ bzw. R⁸ und R⁹ auch durch eine kovalente Bindung verknüpft sein können , so daß eine cyclische Verbindung von vier bis acht Atomen vorliegt,
- X¹ und X²: unabhängig voneinander eine direkte Phosphor-Kohlenstoffbindung, O, S oder NR¹⁰, wobei
- R¹⁰: einem der für R⁶-R⁹ definierten Reste entspricht
- Y¹ und Y²: eine direkte Phosphor-Kohlenstoffbindung, -O-, oder -NR¹¹-darstellt, wobei
- R¹¹: einem der für R⁶-R⁹ definierten Reste entspricht
- Z: miteinander durch Einfach- oder Mehrfachbindung verbundene 1 - 6 Kohlenstoffatome bedeutet, die die Einheit (R⁶Y¹)(R⁷Y¹)PX¹ mit der Einheit X²P(Y²R⁸)(Y²R⁹) verbinden, wobei Z Teil eines gegebenenfalls Heteroatome, bevorzugt aus der Gruppe N, O, S, enthaltenden, aliphatischen, cyloaliphatischen, olefinischen, cycloolefinischen Systems, eines Metallocens, insbesondere ein Ferrocen, ein 1,1'disubstituiertes Ferrocen, 1-(1-Ethylenyl)-2-ferrocenyl oder ein 1,2-disubstituiertes Ferrocen, oder eines oder mehrerer aromatischer oder heteroaromatischer Ringsysteme sein kann, welche gegebenenfalls mit Substituenten wie für R⁶-R⁹ angegeben sowie direkt mit C₁-C₁₀-Alkoxy, OCO-Alkyl-(C₁-C₈), O-Aryl-(C₅-C₁₀), O-Phenyl, C₁-C₉-Trihalomethylalkyl, Trifluormethyl, Trichlormethyl, Fluoro, Chloro, Bromo, lodo, Nitro, Hydroxy, Trifluormethylsulfonato, Oxo, Thio, Thiolato, Amino, C₁-C₈-substituierte Amino der Formen NH₂, NH-Alkyl-C₁-C₈, NH-Aryl-C₅-C₆, N-Alkyl₂-C₁-C₈, N-Aryl₂-C₅-C₆, N-Alkyl₃-C₁-C₈ ⁺, N-Aryl₃-C₅-C₆ ⁺, Cyano, C₁-C₆-Acyloxy, Carboxylato der Formen COOH und COOR¹² Sulfinato, Sulfonato der Formen SO₃H und SO₃ R¹², Phosphato der Formen PO₃H₂, PO₃HR¹² und PO₃R¹² ₂ wobei R¹² entweder ein einwertiges Kation, NH₄ ⁺, N(Alkyl)₄ ⁺, N(Aryl)₄ ⁺, C₁-C₈-Alkyl oder C₆-Aryl darstellt, C₁-C₆-Trialkylsilyl, NHCO-Alkyl-(C₁-C₄), COO-Alkyl-(C₁-C₈), CONH₂, CON(Alkyl-(C₁-C₈))₂, CO-Alkyl-(C₁-C₈), CO-Alkenyl-(C₁-C₈), NHCOO-Alkyl-(C₁-C₄), CO-Aryl-(C₆-C₁₀), CO-Phenyl, COO-Aryl-(C₆-C₁₀), COO-Phenyl, CHCH-CO₂-Alkyl-(C₁-C₈), CHCHCO₂H ein oder mehrfach substituiert sein kann und
- P: ein dreiwertiger Phosphor ist.

In einer bevorzugten Ausführung bedeutet unabhängig voneinander
- R¹ und R²: gleich oder verschieden eine C₁-C₂₀ kohlenstoffhaltige Gruppe wie C₁-C₂₀-Alkyl, C₂-C₈-Alkenyl, C₃-C₇-Cycloalkyl, C₃-C₆-Cycloalkenyl, wobei die Cyclen auch 1 - 2 Heteroatome enthalten können, bevorzugt ausgewählt aus der Gruppe N,O, S und die Ringgröße bevorzugt 5 - 7 beträgt, Phenyl, Naphtyl, Fluorenyl,
wobei diese Gruppen selbst jeweils ein- oder mehrfach substituiert sein können und diese Substituenten dabei unabhängig voneinander Wasserstoff, C₁-C₁₀-Alkyl, C₂-C₁₀-Alkenyl, C₁-C₆-Haloalkyl, C₃-C₇-Cycloalkyl, C₅-C₇-Cycloalkenyl, Phenyl, C₆-C₁₄-Aryl, C₁-C₆-Alkoxy, C₁-C₆-Trihalomethylalkyl, Trifluormethyl, Trichlormethyl, Fluoro, Chloro, Bromo, lodo, Nitro, Trifluormethylsulfonato, C₁-C₄-Trialkylsilyl sein können,
- R³ bis R⁵: gleich oder verschieden ein Wasserstoffatom oder eine C₁-C₂₀ kohlenstoffhaltige Gruppe wie C₁-C₂₀-Alkyl, C₂-C₈-Alkenyl, C₃-C₇-Cycloalkyl, C₃-C₆-Cycloalkenyl, wobei die Cyclen auch 1 - 2 Heteroatome enthalten können, bevorzugt ausgewählt aus der Gruppe N,O, S und die Ringgröße bevorzugt 5 - 7 beträgt, Phenyl, Naphtyl, Fluorenyl, C₂-C₆-Heteroaryl, wobei die Zahl der Heteroatome 1 - 2 betragen kann, bevorzugt ausgewählt aus der Gruppe N,O, S, und die Ringgröße bevorzugt 5 - 7 beträgt,
wobei diese Gruppen selbst jeweils ein- oder mehrfach substituiert sein können und diese Substituenten dabei unabhängig voneinander Wasserstoff, C₁-C₁₀-Alkyl, C₂-C₁₀-Alkenyl, C₁-C₆-Haloalkyl, C₃-C₇-Cycloalkyl, C₅-C₇-Cycloalkenyl, Phenyl, C₃-C₆-Heteroaryl, wobei die Zahl der Heteroatome 1 - 2 betragen kann, bevorzugt ausgewählt aus der Gruppe N,O, S, und die Ringgröße bevorzugt 5 - 7 beträgt, C₁-C₆-Alkoxy, C₁-C₆-Trihalomethylalkyl, Trifluormethyl, Trichlormethyl, Fluoro, Chloro, Bromo, lodo, Nitro, Hydroxy, Trifluormethylsulfonato, Thio, Thiolato, Amino, C₁-C₆-substituierte Amino der Formen NH₂, NH-Alkyl-C₁-C₆, NH-Phenyl, N-Alkyl₂-C₁-C₆, N-Phenyl₂, N-Alkyl₃-C₁-C₆ ⁺, N-Phenyl₃ ⁺, NH-CO-Alkyl-C₁-C₆, NH-CO-Phenyl, Cyano, C₁-C₆-Acyloxy, Sulfinato, Sulfonato der Formen SO₃H und SO₃R¹², Phosphato der Formen PO₃H₂, PO₃HR¹² und PO₃ R¹² ₂ wobei R¹² entweder ein einwertiges Kation, NH₄ ⁺, N(Alkyl)₄ ⁺, N(Phenyl)₄ ⁺, C₁-C₆-Alkyl oder C₆-Aryl darstellt, C₁-C₆-Trialkylsilyl, sein können.

Besonders bevorzugt sind Enamine, bei denen zwei der Substituenten R¹ bis R⁵ durch kovalente Bindungen derart miteinander verknüpft sind, so daß ein fünf- bis siebengliedriger Ring vorliegt.

Bevorzugte Liganden sind Liganden der allgemeinen Formeln (III) und unter denen sind solche bevorzugt, bei denen R⁶ bis R⁹ unabhängig voneinander C₁-C₈-Alkyl, C₅-C₆-Cycloalkyl, C₆-Aryl, C₄-C₅-Heteroaryl, wobei die Zahl der Heteroatome 1 - 2 beträgt, bevorzugt ausgewählt aus der Gruppe N, O, S und die Ringgröße 5 - 6 beträgt, Naphtyl sind, dabei können diese Gruppen einen oder mehrere Substituenten tragen, bevorzugt sind Substituenten, die unabhängig voneinander Wasserstoff, C₁-C₁₀ Alkyl, C₁-C₆ Haloalkyl, C₅-C₆ Cycloalkyl, C₂-C₉ Heterocycloalkyl, C₆Aryl, Phenyl, C₄-C₅ Heteroaryl, wobei die Zahl der Heteroatome, bevorzugt aus der Gruppe N, O, S, 1 - 2 betragen kann, C₁-C₆ Alkoxy, OCO-Alkyl-(C₁-C₆), O-Aryl-C₆, C₁-C₆ Trihalomethylalkyl, Fluoro, Chloro, Bromo, Iodo, Nitro, Hydroxy, Trifluormethylsulfonato, Oxo, Thio, Thiolato, Amino, C₁-C₈ substituierte Amino der Formen mono-, di-, tri- C₁-C₈-Alkylamino oder C₂-C₈ Alkenylamino oder mono-, di-, tri- C₆-C₈ Arylamino oder C₁-C₈-Alkyl-C₆-C₈-arylamino, NH-CO-Alkyl- C₁-C₈, NH-CO-Aryl- C₆-C₈, Cyano, C₁-C₈-Acyloxy, Carboxyl, Carboxylato der Form COOR¹², Sulfinato, Sulfonato der Form SO₃R¹², Phosphato der Form PO₃H₂, PO₃HR¹², PO₃R¹² ₂, wobei wobei R¹² entweder ein einwertiges Kation, NH₄ ⁺, N(Alkyl)₄ ⁺, N(Aryl)₄ ⁺, C₁-C₈-Alkyl oder C₆-Aryl darstellt, Tri- C₁-C₆ Alkylsilyl, sein,
bevorzugt sind Liganden, bei denen Y¹ und Y² eine direkte Phosphor-Kohlenstoffbindung darstellen, und bei denen Z aus ein bis vier Kohlenstoffatomen, besonders bevorzugt aus zwei Kohlenstoffatomen, besteht.
Besonders bevorzugt sind Systeme, bei denen aus Z, X¹ ,X², P¹ und P² mit einem koordinierenden Metall ein Siebenring gebildet werden kann.

Ist Z Teil eines cyclischen Strukturelementes, sind drei bis neungliedrige Ringsysteme bevorzugt. Besonders bevorzugt sind fünf bis siebengliedrige Ringsysteme. Das Ringsystem kann ein bis vier Heteroatome enthalten, bevorzugt ein bis zwei. Bevorzugt sind dabei O, N und S. Der Schwefel S kann in verschiedenen Oxidationsformen vorliegen, bevorzugt neben -S- ist --SO₂. Der Stickstoff des Ringsystems kann als NR, NR₂ ⁺, NRH⁺, NC(O)R, NSO₂R, NP(O)R₂ vorliegen. Die Ringsysteme können wie für R⁶ bis R⁹ angegeben oder mit Alkoxy, Halogeno, Nitro, Hydroxy, Oxo, Thio, Thiolato, Amino, substituierten Amino, Cyano, Sulfonato, Phosphonato,Trialkylsilylgruppen ein oder mehrfach direkt substituiert sein, wobei die Substituenten auch untereinander verbrückt sein können. Besonders bevorzugte Ringsysteme sind unsubstituierte oder wie vorstehend angegeben substituierte Phenyl, Ferrocenyl, Cyclopentyl, Cyclohexyl, Pyridyl, Pyrrol, Furyl, Thiophen, Tetrahydrofuran, Tetrahydrothiophen, Piperidyl, Pyrrolidinyl, Ferrocenyl, Dioxolan oder Sulfolanringe.

Metallocene wie Ferrocene sollen nach dem Verständnis dieser Erfindung formal zur Gruppe der Aromaten gerechnet werden.

Vorzugsweise weist das erfindungsgemäße Ligandsystem in R¹-R¹² Alkyl, Alkenyl, Cycloalkyl, Cycloalkenyl, Alkoxy, Trialkylsilyl oder/und Dialkylaminogruppen unabhängig voneinander auf, die jeweils 1 bis 20, insbesondere 1 bis 6 Kohlenstoffatome enthalten.

Aus der Gruppe der Alkylsubstituenten seien bevorzugt genannt Methyl, Ethyl, n-Propyl, 1-Methylethyl, n-Butyl, 1-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, n-Heptyl, n-Octyl, n-Nonyl, n-Decyl.

Unter den cyclischen Alkylsubstituenten sind besonders bevorzugt substituierte und unsubstituierte Cyclopentyl, Cyclohexyl, Cycloheptyl.

Als Alkenylreste seien bevorzugt Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 1-Heptenyl, 2-Heptenyl, 1-Octenyl oder 2-Octenyl genannt.

Unter den cyclischen Alkenylsubstituenten sind besonders bevorzugt Cyclopentenyl, Cyclohexenyl, Cycloheptenyl und Norbornyl.

Unter Arylsubstituenten in R¹-R¹² sind besonders bevorzugt Phenyl, 2-Alkylphenyl, 3-Alkylphenyl, 4-Alkylphenyl, 2,6-Dialkylphenyl, 3,5-Dialkylphenyl, 3,4,5-Trialkylphenyl, 2-Alkoxyphenyl, 3-Alkoxyphenyl, 4-Alkoxyphenyl, 2,6-Dialkoxylphenyl, 3,5-Dialkoxyphenyl, 3,4,5-Triialkoxyphenyl, 3,5-Dialkyl-4-Alkoxyphenyl, 3,5-Dialkyl-4-dialkylaminophenyl, 4-Dialkylamino, wobei die vorgenannten Alkyl und Alkoxygruppen jeweils vorzugsweise 1 - 6 Kohlenstoffatome enthalten,
3,5-Trifluormethyl, 4-Trifluormethyl, 2-Sulfonyl, 3-Sulfonyl, 4-Sulfonyl, ein bis vierfach halogenierte Phenyl, Fluorenyl und Naphtyl. Bevorzugte Halogene sind F, Cl und Br.

Alle Haloalkyl- oder/und Haloarylgruppen weisen vorzugsweise die allgemeinen Formeln CHal₃, CH₂CHal₃, C₂Hal₅ auf, wobei Hal insbesondere für F, Cl und Br stehen kann. Besonders bevorzugt sind Haloalkyl- oder/und Haloarylgruppen der Formeln CF₃, CH₂CF₃, C₂F₅.

Typische Vertreter der im erfindungsgemäßen Verfahren verwendeten Ligandsysteme sind Diphosphinit-Liganden auf Kohlenhydratbasis wie z. B. in DD 140036, DD 240372, DD 248 028 und WO 95/18787 beschrieben und verwandte Ligandsysteme wie z. B. das bdpch (1,2-Cyclohexyldiarylphosphinit), Aminophosphin-Phosphinite (Agbossou et al., Coordination Chemistry Rev. 1998, 178-180, 1615), DIOP- (2,3-O-isopropyliden-2,3-dihydroxy-1,4-bis(diphenylphosphino)butane)- und modifizierte DIOP-Liganden (Kagan et al. J. Amer. Chem. Soc. 1972, 946429; Achiwa et. al. Chem. Pharm. Bull, 1993, 41, 1149), BPPM {N-(t-butoxycarbonyl)-4-(diphenylphosphino)-2-[(diphenylphosphino)methyl]pyrrolidin} (Achiwa, J. Amer. Chem. soc. 1976, 98, 8265) und Modifikationen dieses Ligandentypes.
Die phosphorhaltigen Liganden lassen sich unter den dem Fachmann geläufigen Bedingungen herstellen (beispielsweise nach Methoden wie sie in Chemistry of Organophosphorous Compounds, Ed. F. R. Hartley, Serial Ed. S. Patai, Vol. 1, John Whiley, 1990 beschrieben sind). Die Liganden und oder Metallkomplexe sind teilweise auch kommerziell erhältlich (beispielsweise von Aldrich oder Strem/ABCR).

Die Liganden der allgemeinen Formeln (III) und (IV) bilden unter anderem Komplexverbindungen vom Typ (V), die als Katalysatoren bei der erfindungsgemäßen asymmetrischen Hydrierung von elektronenreichen Enaminen wirken,

[MₓPₘLₙS_{q}]Aᵣ (V)

wobei in der allgemeinen Formel (V) M ein Metallzentrum ist und mindestens ein solches Zentrum ein Metallatom ausgewählt aus der 8. Nebengruppe, bevorzugt Rhodium oder Iridium, besonders bevorzugt Rhodium, enthält, L gleiche oder verschiedene koordinierende organische oder anorganische Liganden und P organische Liganden der Formeln (III) und/oder (IV) darstellen, S koordinierende Lösungsmittelmoleküle und A Äquivalente aus nicht koordinierenden Anionen repräsentiert, wobei x und m ganzen Zahlen größer oder gleich 1, n, q und r ganze Zahlen größer oder gleich 0 sind.

Die Summe m + n + q wird durch die an den Metallzentren zur Verfügung stehenden Koordinationszentren nach oben begrenzt, wobei nicht alle Koordinationsstellen besetzt sein müssen. Bevorzugt sind Komplexverbindungen mit oktaedrischer, pseudo-oktaedrischer, tetraedrischer, pseudo-tetraedrischer, quadratisch-planarer Koordinationssphäre, die auch verzerrt sein kann, um das jeweilige Übergangsmetallzentrum. Die Summe m + n + q ist in solchen Komplexverbindungen kleiner oder gleich 6x.

Bevorzugt sind Komplexverbindungen mit weniger als vier Metallzentren, besonders bevorzugt solche mit ein oder zwei Metallzentren.
Bevorzugte Liganden L solcher Komplexverbindungen sind Halogenid, besonders Cl, Br und 1, Dien, besonders Cyclooctadien, Norbornadien, Olefin, besonders Ethylen und Cycloocten, Acetato, Trifluoracetato, Acetylacetonato, sowie Carbonyl und Hydrido Liganden.

Bevorzugte koordinierende Lösungsmittel S sind Amine, besonders Triethylamin, Alkohole, besonders Methanol und Aromaten, besonders Benzol und Cumol.

Bevorzugte nichtkoordinierende Anionen A sind Trifluorsulfonat, BF₄; ClO₄, PF₆, und BAr₄.

In den einzelnen Komplexverbindungen können dabei unterschiedliche Moleküle, Atome oder Ionen der einzelnen Bestandteile M, P, L, S und A enthalten sein.

Bevorzugt unter den ionisch aufgebauten Komplexverbindungen sind Verbindungen des Typs [RhPₘ(Dien)]⁺A⁻, wobei Pₘ entweder zwei monodentate Liganden der Formel (IV) oder einen bidentaten Liganden der Formel (III) repräsentiert.

Besonders bevorzugt als Katalysatoren in dieser Erfindung sind
Komplexverbindungen aus Liganden der Formel (III), die Rhodium als Zentralatom enthalten. Unter diesen sind besonders ionische Komplexverbindungen des Typs [RhPₘ(Dien)]⁺A⁻ bevorzugt, wobei Pₘ einen bidentaten Liganden der Formel (III) repräsentiert.

Die Herstellung dieser Metall-Ligand-Komplexverbindungen kann in situ durch Reaktion eines Metallsalzes oder eines entsprechenden Vorkomplexes mit den Liganden der allgemeinen Formeln (III) und/oder (IV) erfolgen. Darüber hinaus kann eine Metall-Ligand-Komplexverbindung durch Reaktion eines Metallsalzes oder eines entsprechenden Vorkomplexes mit den Liganden der allgemeinen Formel (III) und/oder (IV) und anschließende Isolierung gewonnen werden.

Beispielsweise können Metallkomplexe synthetisiert werden, indem man in bekannter Weise (EP-A -0158875; EP-A-0437690) durch Umsetzung von Rhodium-oder, Iridiumkomplexen, die labile Liganden, wie z. B. [Rh(COD)₂]BF₄, RuCl₂(COD)]ₙ, [lr(COD)CI]₂ enthalten, mit den phosphorhaltigen Liganden der Formeln (III) und/oder (IV) katalytisch aktive Komplexe generiert. Darüber hinaus können dem Metallorganiker geläufigen Methoden zur Generierung entsprechender Komplexe genutzt werden (Comprehensive Organometallic Chemistry II, Pergamon 1995). Die Katalysatoren können dabei in situ aus dem Vorkomplex und dem Liganden erzeugt werden, oder sie werden in isolierter Form eingesetzt.

Beispiele für die Metallsalze sind Metallchloride, -bromide, -iodide, -nitrate, -acetate, -acetylacetonate, -hexafluoracetylacetonate, -perfluoracetate oder -triflate des Rhodiums und Iridiums.

Beispiele für die Vorkomplexe sind:
Cyclooctadienrhodium(l)chlorid-Dimer, Norbornadienrhodium(I)chlorid-Dimer,
1,5-Hexadienrhodium(I)chlorid-Dimer, Tris(triphenylphosphan)rhodium(I)chlorid,
Hydridocarbonyltris(triphenylphosphan)rhodium(I)chlorid,
Bis(cyclooctadien)rhodium(l)perchlorat, Bis(cyclooctadien)rhodium(I)tetrafluorborat,
Bis(cyclooctadien)rhodium(I)triflat, Bis(acetonitrilcyclooctadien)rhodium(I)perchlorat,
Bis(acetonitrilcyclooctadien)rhodium(I)tetrafluorborat,
Bis(acetonitrilcyclooctadien)rhodium(I)triflat,
Cyclopentadienrhodium(III)chlorid-Dimer,
Pentamethylcyclopentadienrhodium(lll)chlorid-Dimer,
Cyclooctadieniridium(I)chlorid-Dimer, Bis(cycloocten)iridium(l)chlorid-Dimer.

Die erfindungsgemäße asymmetrische Hydrierung wird in der Regel bei einer Temperatur von -20 -100 °C, bevorzugt bei 0 - 50 °C, besonders bevorzugt bei Raumtemperatur durchgeführt.

Der Wasserstoffanfangsdruck kann in einem großen Bereich zwischen 0.1 bar und 300 bar für das erfindungsgemäße Verfahren variiert werden. Sehr gute Ergebnisse erhält man im Druckbereich von 1 - 60 bar.

Die Umsetzung wird bevorzugt in homogener Lösung durchgeführt. Insbesondere inerte Lösungsmittel haben sich als vorteilhaft erwiesen. Bevorzugte Lösungsmittel für die reduktive Aminierung sind C1-C4-Alkanole, insbesondere MeOH, aber auch Wasser. Bei schlecht löslichen Substraten sind auch Lösungsmittelgemische, z. B. MeOH und CH₂C₁₂ oder THF, Toluol geeignet.

Vorteilhaft im Sinne dieser Erfindung ist, daß die Herstellung einiger Enamine aus den leicht zugänglichen, aber nicht einfach zu hydrierenden Iminen durch Alkylierung und basenkatalysierte Umlagerung erfolgen kann.

Der Katalysator wird üblicherweise in Mengen von 0.001 bis 5 mol-%, bevorzugt 0.001 bis 1 mol-%, bezogen auf das Enamin der Formel (I) eingesetzt.

Die nachstehenden Beispiele dienen der Erläuterung der Erfindung, ohne diese darauf zu beschränken.

### Beispiel 1:

In einem Autoklaven wurde eine Lösung von 1.0 mmol 2-*N*-Piperidinylstyrol und 0.01 mmol {Rh[(*S,S*)-bppm](COD)}BF₄ in 15 ml MeOH bei Raumtemperatur und einem Anfangswasserstoffdruck von 50 bar 18 h lang gerührt. Unter diesen Bedingungen wurde das Enamin quantitativ zum Amin hydriert. (*R*)-2-*N*-Piperidinylethylbenzol wurde in 50 %ee erhalten.

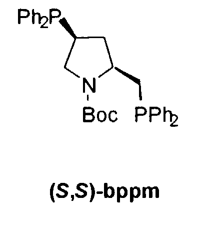

### Beispiel 2 :

In einem Autoklaven wurde eine Lösung von 1.0 mmol 2-*N*-Piperidinylstyrol und 0.01 mmol {Rh[(*R,R*)-DIOP](COD)}BF₄ in 15 ml MeOH bei Raumtemperatur und einem Anfangswasserstoffdruck von 50 bar 2 h lang gerührt. Unter diesen Bedingungen wurde das Enamin quantitativ zum Amin hydriert. (*R*)-2-*N*-Piperidinylethylbenzol wurde in 32 %ee erhalten.

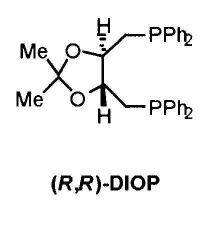

### Beispiel 3 :

In einer Hydrierapparatur wurde eine Lösung von 1.0 mmol 2-*N*-Piperidinylstyrol und 0.01 mmol {Rh[(*R,R*)-DIOP](COD)}BF₄ in 15 ml MeOH bei Raumtemperatur und einem Anfangswasserstoffdruck von 1 bar 2 h lang gerührt. Unter diesen Bedingungen wurde das Enamin quantitativ zum Amin hydriert. (*R*)-2-*N*-Piperidinylethylbenzol wurde in 39 %ee erhalten.

### Tabelle 1:

Übersicht über die asymmetrische Hydrierung von 2-*N*-Piperidinylstyrol mit Diphosphin- bzw. Diphosphinitliganden^{a}

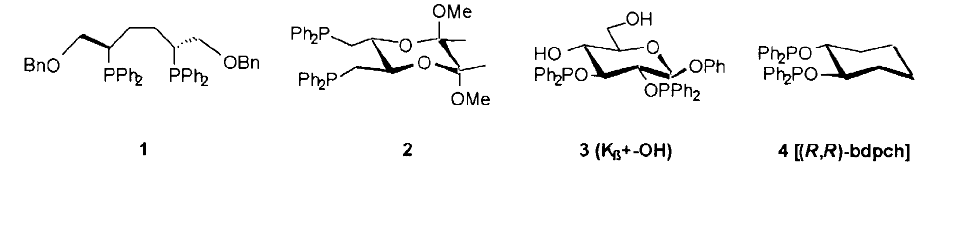

| **Beispiel** | **Katalysator** | **Zeit (h)** | **Umsatz (%)** | **ee (%)** |
|---|---|---|---|---|
| 4 | [Rh(**1**)(COD)]BF₄ | 10 | > 99 | 53 *(R)* |
| 5 | [Rh(**2**)(COD)]BF₄ | 2 | > 99 | 45 *(R)* |
| 6 | [Rh(**3**)(COD)]BF₄ | 28 | > 99 | 36 *(R)* |
| 7 | [Rh(**4**)(COD)]BF₄ | 3 | > 99 | 32 (*R*) |
| ^{a} Bedingungen: 1.0 mmol Substrat, 0.01 mmol Präkatalysator, 15 ml MeOH, RT, Anfangswasserstoffdruck: 1 bar. | | | | |

### Beispiel 8:

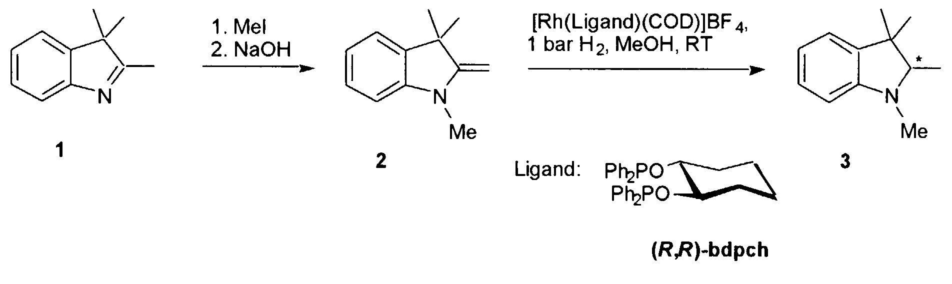

2,3,3-Trimethylindolenin **1** wurde durch *N*-Alkylierung mit Methyliodid und nachfolgender basischer Umlagerung in das entsprechende *N*-Methyl-enamin **2** umgewandelt. Anschließend wurde eine Lösung von 1.0 mmol des Enamins **2** in 15 ml MeOH in einer Hydrierapparatur mit 0.01 mmol {Rh[(*R,R*)-bdpch](COD)}BF₄ bei Raumtemperatur und einem Anfangswasserstoffdruck von 1 bar 4 h lang gerührt. Unter diesen Bedingungen wurde das Enamin quantitativ zum Amin **3** hydriert. Das Amin wurde in 70 %ee erhalten.

### Beispiel 9 :

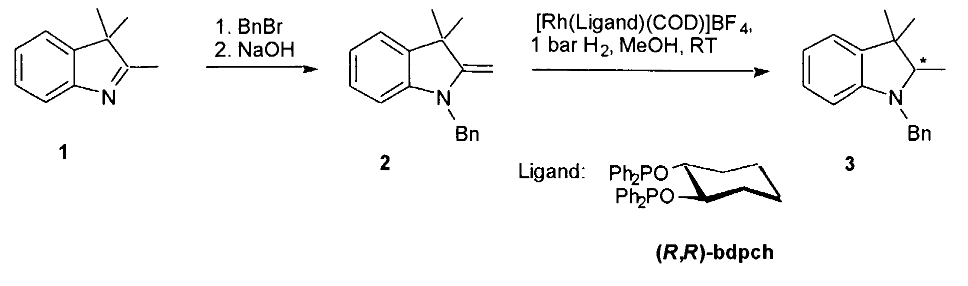

2,3,3-Trimethylindolenin **1** wurde durch *N*-Alkylierung mit Benzylbromid und basischer Umlagerung in das entsprechende *N*-Benzyl-enamin **2** umgewandelt. Anschließend wurde eine Lösung von 1.0 mmol des Enamins **2** in 15 ml MeOH in einer Hydrierapparatur mit 0.01 mmol {Rh[(*R,R*)-bdpch](COD)}BF₄ bei Raumtemperatur und einem Anfangswasserstoffdruck von 1 bar 4 h lang gerührt. Unter diesen Bedingungen wurde das Amin **3** mit 86 % Ausbeute und in 67 %ee erhalten.

### Beispiel 10 :

2,3,3-Trimethylindolenin wurde durch *N*-Alkylierung mit Methyliodid in das entsprechende *N*-Methyl-enamin umgewandelt. Anschließend wurde eine Lösung von 1.0 mmol des Enamins in 10 ml MeOH in einem Autoklaven mit 0.002 mmol {Rh[(*R,R*)-bdpch](COD)}BF₄ bei Raumtemperatur und einem Anfangswasserstoffdruck von 50 bar 0.5 h lang gerührt. Unter diesen Bedingungen wurde das gewünschte Amin quantitativ und in 72 %ee erhalten.

### Beispiel 11 :

2,3,3-Trimethylindolenin wurde durch *N*-Alkylierung mit Benzylbromid in das entsprechende *N*-Benzyl-enamin umgewandelt. Anschließend wurde eine Lösung von 1.0 mmol des Enamins **2** in 10 ml MeOH in einem Autoklaven mit 0.002 mmol {Rh[(*R,R*)-bdpch](COD)}BF₄ bei Raumtemperatur und einem Anfangswasserstoffdruck von 50 bar 0.5 h lang gerührt. Unter diesen Bedingungen wurde das gewünschte Amin quantitativ und in 67 %ee erhalten.

## Patentansprüche

1. Verfahren zur Herstellung von chiralen Aminen durch asymmetrische Hydrierung von Enaminen dadurch gekennzeichnet, daß elektronenreiche Enaminen in Gegenwart von Wasserstoff und eines chiralen Katalysators umfassend mindestens einen Metall-Ligand-Komplex, der mindestens ein Metallatom ausgewählt aus der 8. Nebengruppe und ein oder mehrere mono-oder bidentate Liganden der allgemeinen Formeln (III) oder (IV) enthält,
(R⁶Y¹)(R⁷Y¹)P¹X¹ZX²P²(Y²R⁸)(Y²R⁹) (III)
(R⁶Y¹)(R⁷Y¹)(R⁸Y¹)P (IV)
wobei
R⁶ bis R⁹ gleich oder verschieden sind und ein Wasserstoffatom oder eine C₁-C₅₀ kohlenstoffhaltige Gruppe, wie z. B. C₁-C₂₄Alkyl, C₂-C₂₀ Alkenyl, C₃-C₈Cycloalkyl, C₅-C₈ Cycloalkenyl, C₆-C₁₄ Aryl, Phenyl, Naphthyl, Fluorenyl, C₂-C₁₃ Heteroaryl, wobei die Zahl der Heteroatome 1 - 4 betragen kann,
und bei denen alle vorgenannten Substituenten jeweils ein- oder mehrfach substituiert sein können, diese Substituenten können dabei unabhängig voneinander Wasserstoff, C₁-C₂₀ Alkyl, C₂-C₂₀ Alkenyl, C₁-C₁₀ Haloalkyl, C₃-C₈ Cycloalkyl, C₅-C₈ Cycloalkenyl, C₂-C₉ Heterocycloalkyl, C₁-C₉ Heterocycloalkenyl, C₆-C₁₄Aryl, Phenyl, C₂-C₁₃ Heteroaryl, wobei die Zahl der Heteroatome 1 - 4 betragen kann, C₁-C₁₀ Alkoxy, OCO-Alkyl-(C₁-C₈), O-Aryl-(C₅-C₁₀), O-Phenyl, C₁-C₉ Trihalomethylalkyl, Fluoro, Chloro, Bromo, lodo, Nitro, Hydroxy, Trifluormethylsulfonato, Oxo, Thio,
Thiolato, Amino, C₁-C₈ substituierte Amino der Formen mono-, di-, tri- C₁-C₈-Alkylamino oder C₂-C₈ Alkenylamino oder mono-, di-, tri- C₆-C₈ Arylamino oder C₁-C₈-Alkyl-C₆-C₈-arylamino, NH-CO-Alkyl-C₁-C₈, NH-CO-Aryl-C₆-C₈, Cyano, C₁-C₈-Acyloxy, Carboxyl, Carboxylato der Form COOR¹², Sulfinato, Sulfonato der Form SO₃R¹², Phosphato der Form PO₃H₂, PO₃HR¹², PO₃R¹² ₂, wobei R¹² entweder ein einwertiges Kation, NH₄ ⁺, N(Alkyl)₄ ⁺, N(Aryl)₄ ⁺, C₁-C₈-Alkyl oder C₆-Aryl darstellt, Tri- C₁-C₆ Alkylsilyl, sein,
und wobei zwei dieser Substituenten auch verbrückt sein können, und R⁶ und R⁷ bzw. R⁸ und R⁹ auch durch eine kovalente Bindung verknüpft sein können , so daß eine cyclische Verbindung von vier bis acht Atomen vorliegt,
X¹ und X² unabhängig voneinander eine direkte Phosphor-Kohlenstoffbindung, O, S oder NR¹⁰, wobei
R¹⁰ einem der für R⁶-R⁹ definierten Reste entspricht
Y¹ und Y² eine direkte Phosphor-Kohlenstoffbindung, -O-, oder -NR¹¹-darstellt, wobei
R¹¹ einem der für R⁶-R⁹ definierten Reste entspricht
Z miteinander durch Einfach- oder Mehrfachbindung verbundene 1 - 6 Kohlenstoffatome bedeutet, die die Einheit (R⁶Y¹)(R⁷Y¹)PX¹ mit der Einheit X²P(Y²R⁸)(Y²R⁹) verbinden, wobei Z Teil eines gegebenenfalls Heteroatome enthaltenden, aliphatischen, cyloaliphatischen, olefinischen, cycloolefinischen Systems, eines Metallocens, insbesondere ein Ferrocen, ein 1,1'disubstituiertes Ferrocen, 1-(1-Ethylenyl)-2-ferrocenyl oder ein 1,2-disubstituiertes Ferrocen, oder eines oder mehrerer aromatischer oder heteroaromatischer Ringsysteme sein kann, welche gegebenenfalls mit Substituenten wie für R⁶-R⁹ angegeben sowie direkt mit C₁-C₁₀-Alkoxy, OCO-Alkyl-(C₁-C₈), O-Aryl-(C₅-C₁₀), O-Phenyl, C₁-C₉-Trihalomethylalkyl, Trifluormethyl, Trichlormethyl, Fluoro, Chloro, Bromo, lodo, Nitro, Hydroxy, Trifluormethylsulfonato, Oxo, Thio, Thiolato, Amino, C₁-C₈-substituierte Amino der Formen NH₂, NH-Alkyl-C₁-C₈, NH-Aryl-C₅-C₆, N-Alkyl₂-C₁-C₈, N-Aryl₂-C₅-C₆, N-Alkyl₃-C₁-C₈ ⁺, N-Aryl₃-C₅-C₆ ⁺, Cyano, C₁-C₆-Acyloxy, Carboxylato der Formen COOH und COOR¹² Sulfinato, Sulfonato der Formen SO₃H und SO₃ R¹², Phosphato der Formen PO₃H₂, PO₃HR¹² und PO₃R¹² ₂ wobei R¹² entweder ein einwertiges Kation, NH₄ ⁺, N(Alkyl)₄ ⁺, N(Aryl)₄ ⁺, C₁-C₈-Alkyl oder C₆-Aryl darstellt, C₁-C₆-Trialkylsilyl, NHCO-Alkyl-(C₁-C₄), COO-Alkyl-(C₁-C₈), CONH₂, CON(Alkyl-(C₁-C₈))₂, CO-Alkyl-(C₁-C₈), CO-Alkenyl-(C₁-C₈), NHCOO-Alkyl-(C₁-C₄), CO-Aryl-(C₆-C₁₀), CO-Phenyl, COO-Aryl-(C₆-C₁₀), COO-Phenyl, CHCH-CO₂-Alkyl-(C₁-C₈), CHCHCO₂H ein oder mehrfach substituiert sein kann und
P ein dreiwertiger Phosphor ist,
umgesetzt werden.

2. Verfahren nach Anspruch 1 dadurch gekennzeichnet, daß Z Teil eines drei bis neungliedrige Ringsysteme, bevorzugt eines fünf bis siebengliedrigen Ringsystems ist, wobei das Ringsystem ein bis vier Heteroatome, bevorzugt ein bis zwei, enthalten kann.

3. Verfahren nach Anspruch 2 dadurch gekennzeichnet, daß die Heteroatome aus der Gruppe O, N und S ausgewählt sind, wobei der Schwefel S in verschiedenen Oxidationsformen, bevorzugt als -S- oder -SO₂-, und der Stickstoff als NR, NR₂ ⁺, NRH⁺, NC(O)R, NSO₂R, NP(O)R₂ vorliegen kann.

4. Verfahren nach einem der Ansprüche 2 oder 3 dadurch gekennzeichnet, daß Z Teil eines unsubstituierte oder substituierte Phenyl, Ferrocenyl, Cyclopentyl, Cyclohexyl, Pyridyl, Pyrrol, Furyl, Thiophen, Tetrahydrofuran, Tetrahydrothiophen, Piperidyl, Pyrrolidinyl, Ferrocenyl, Dioxolan oder Sulfolanringe Ringsysytems ist.

5. Verfahren nach einem der vorherigen Ansprüche dadurch gekennzeichnet, daß ein chiraler Katalysator der Formel (V) verwendet wird,
[MₓPₘLₙS_{q}]Aᵣ (V)
wobei
M ein Metallzentrum ist und mindestens ein solches Zentrum ein Metallatom ausgewählt aus der 8. Nebengruppe enthält,
L gleiche oder verschiedene koordinierende organische oder anorganische Liganden und
P organische Liganden der Formeln (III) und/oder (IV) darstellen,
S koordinierende Lösungsmittelmoleküle und
A Äquivalente aus nicht koordinierenden Anionen repräsentiert,
wobei x und m ganzen Zahlen größer oder gleich 1, n, q und r ganze Zahlen größer oder gleich 0 sind und die Summe m + n + q durch die an den Metallzentren zur Verfügung stehenden Koordinationszentren nach oben begrenzt ist, wobei nicht alle Koordinationsstellen besetzt sein müssen.

6. Verfahren nach Anspruch 5 dadurch gekennzeichnet, daß Katalysatoren mit weniger als vier Metallzentren, besonders bevorzugt solche mit ein oder zwei Metallzentren verwendet werden.

7. Verfahren nach einem der vorherigen Ansprüche dadurch gekennzeichnet, daß der Katalysator mindestens ein Rhodium- oder Iridiumatom enthält.

8. Verfahren nach Anspruch 7 dadurch gekennzeichnet, daß der Katalysator vom Typ [RhPₘ(Dien)]⁺A⁻ ist, wobei Pₘ entweder zwei monodentate Liganden der Formel (IV) oder einen bidentaten Liganden der Formel (III) repräsentiert.

9. Verfahren nach einem der vorherigen Ansprüche dadurch gekennzeichnet, daß als Edukt elektronenreiche Enamine der Formel (I) eingesetzt werden, 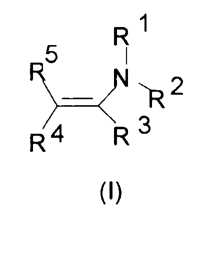 worin
R¹ und R² gleich oder verschieden Wasserstoff, eine C₁-C₅₀ kohlenstoffhaltige Gruppe wie C₁-C₂₄-Alkyl, C₂-C₂₀-Alkenyl, C₃-C₈-Cycloalkyl, C₅-C₈-Cycloalkenyl, wobei der Cyclus auch 1-2 Heteroatome enthalten kann, bevorzugt ausgewählt aus der Gruppe N,O, S und die Ringgröße bevorzugt 5 - 7 beträgt, Phenyl, Naphtyl, Fluorenyl, C₆-C₁₄-Aryl, C₂-C₁₃-Heteroaryl, wobei die Zahl der Heteroatome 1 - 4 betragen kann, bevorzugt ausgewählt aus der Gruppe N,O, S, und die Ringgröße bevorzugt 5 - 7 beträgt,
wobei diese Gruppen selbst jeweils ein- oder mehrfach substituiert sein können und diese Substituenten dabei unabhängig voneinander Wasserstoff, C₁-C₂₀-Alkyl, C₂-C₂₀-Alkenyl, C₁-C₁₀-Haloalkyl, C₃-C₈-Cycloalkyl, C₃-C₈-Cycloalkenyl, Phenyl, C₆-C₁₄-Aryl, C₂-C₁₃-Heteroaryl, wobei die Zahl der Heteroatome 1 - 4 betragen kann, bevorzugt ausgewählt aus der Gruppe N,O, S, und die Ringgröße bevorzugt 5 - 7 beträgt, C₁-C₁₀-Alkoxy, C₁-C₉-Trihalomethylalkyl, Trifluormethyl, Trichlormethyl, Fluoro, Chloro, Bromo, lodo, Nitro, Trifluormethylsulfonato, C₁-C₆-Trialkylsilyl sein können,
R³ bis R⁵ gleich oder verschieden ein Wasserstoffatom oder ein Halogen (Fluor, Chlor, Brom, Jod) oder eine C₁-C₅₀ kohlenstoffhaltige Gruppe wie C₁-C₂₄-Alkyl, C₂-C₂₀-Alkenyl, C₃-C₈-Cycloalkyl, C₃-C₈-Cycloalkenyl, wobei der Cyclus auch 1 - 2 Heteroatome enthalten kann, bevorzugt ausgewählt aus der Gruppe N,O, S und die Ringgröße bevorzugt 5 - 7 beträgt, Phenyl, Naphtyl, Fluorenyl, C₆-C₁₄-Aryl, C₂-C₁₃-Heteroaryl, wobei die Zahl der Heteroatome 1 - 4 betragen kann, bevorzugt ausgewählt aus der Gruppe N,O, S, und die Ringgröße bevorzugt 5 - 7 beträgt, wobei diese Gruppen selbst jeweils ein- oder mehrfach substituiert sein können und diese Substituenten dabei unabhängig voneinander Wasserstoff, C₁-C₂₀-Alkyl, C₂-C₂₀-Alkenyl, C₁-C₁₀-Haloalkyl, C₃-C₈-Cycloalkyl, C₃-C₈-Cycloalkenyl, wobei der Cyclus auch 1 - 2 Heteroatome enthalten kann, bevorzugt ausgewählt aus der Gruppe N,O, S und die Ringgröße bevorzugt 5 - 7 beträgt, Phenyl, C₆-C₁₄-Aryl, C₃-C₁₃-Heteroaryl, wobei die Zahl der Heteroatome 1 - 4 betragen kann, bevorzugt ausgewählt aus der Gruppe N,O, S, und die Ringgröße bevorzugt 5 - 7 beträgt, C₁-C₁₀-Alkoxy, C₁-C₉-Trihalomethylalkyl, Trifluormethyl, Trichlormethyl, Fluoro, Chloro, Bromo, lodo, Nitro, Hydroxy, Trifluormethylsulfonato, Thio, Thiolato, Amino, C₁-C₈-substituierte Amino der Formen NH₂, NH-Alkyl-C₁-C₈, NH-Aryl-C₅-C₆, N-Alkyl₂-C₁-C₈, N-Aryl₂-C₅-C₆, N-Alkyl₃-C₁-C₈ ⁺, N-Aryl₃-C₅-C₆ ⁺, NH-CO-Alkyl-C₁-C₈, NH-CO-Aryl-C₅-C₆, Cyano, C₁-C₆-Acyloxy, Sulfinato, Sulfonato der Formen SO₃H und SO₃R¹², Phosphato der Formen PO₃H₂, PO₃HR¹² und PO₃ R¹² ₂ wobei R¹² entweder ein einwertiges Kation, NH₄ ⁺, N(Alkyl)₄ ⁺, N(Aryl)₄ ⁺, C₁-C₈-Alkyl oder C₆-Aryl darstellt, C₁-C₆-Trialkylsilyl, sein können, und
dabei können R¹ bis R⁵ durch eine und oder mehrere kovalente Bindungen verknüpft sein, so daß ein cyclisches System vorliegt,

10. Verfahren nach einem der vorherigen Ansprüche dadurch gekennzeichnet, daß die Umsetzung bei einer Temperatur von -20 -100 °C, bevorzugt bei 0-50 °C, besonders bevorzugt bei Raumtemperatur durchgeführt wird.

11. Verfahren nach einem der vorherigen Ansprüche dadurch gekennzeichnet, daß der Wasserstoffanfangsdruck zwischen 0.1 bar und 300 bar, bevorzugt im Druckbereich von 1 - 60 bar liegt.

12. Verfahren nach einem der vorherigen Ansprüche dadurch gekennzeichnet, daß die Umsetzung in homogener Lösung erfolgt.

13. Verfahren nach einem der vorherigen Ansprüche dadurch gekennzeichnet, daß der Katalysator in einer Menge von 0.001 bis 5 mol-%, bevorzugt 0.001 bis 1 mol-%, bezogen auf das Enamin der Formel (I) eingesetzt wird.
